Europäisches Patentamt

European Patent Office

Office européen des brevets

(11) Veröffentlichungsnummer: **0 224 153**
**A2**

(12) # EUROPÄISCHE PATENTANMELDUNG

(21) Anmeldenummer: 86115863.2

(22) Anmeldetag: 14.11.86

(51) Int. Cl.⁴: **A61F 2/24**

(30) Priorität: 23.11.85 DE 3541478

(43) Veröffentlichungstag der Anmeldung:
03.06.87 Patentblatt 87/23

(84) Benannte Vertragsstaaten:
**AT BE CH DE FR GB IT LI NL SE**

(71) Anmelder: **Beiersdorf Aktiengesellschaft**
**Unnastrasse 48**
**D-2000 Hamburg 20(DE)**

(72) Erfinder: **Pletsch, Hanns, Dr.**
**Mittelweg 25**
**D-2000 Hamburg 13(DE)**
Erfinder: **Holtzmann, Hans-Joachim**
**Borchertring 64**
**D-2000 Hamburg 60(DE)**
Erfinder: **Sachau, Günther**
**Lessingstrasse 21**
**D-2085 Quickborn(DE)**
Erfinder: **Kartheus, Holger**
**Stellinger Weg 19**
**D-2000 Hamburg 20(DE)**
Erfinder: **Reul, Helmut, Dr.**
**Akazienstrasse 65**
**D-5160 Düren(DE)**

(54) Herzklappenprothese und Verfahren zu deren Herstellung.

(57) Herzklapenprothese zum Ersatz der Aorten-oder Pulmonalklappe aus einem Stützring mit mindestens je zwei Kommisurenstützen und flexiblen Segeln, die dadurch gekennzeichnet ist, daß die Höhe des Stützrings einschließlich der Kommissurenstützen kleiner als die Gesamthöhe der Herzklappenprothese ist.

Fig.1

EP 0 224 153 A2

## Herzklappenprothese und Verfahren zu deren Herstellung

Die Erfindung betrifft eine Herzklappenprothese aus einem Stützring mit mindestens zwei, vorzugsweise drei, Kommissurenstützen und flexiblen Segeln (oder Taschen) sowie deren bevorzugte Herstellungsverfahren. Derartige Prothesen werden zum Ersatz der Aorten-oder Pulmonalklappen am menschlichen Herzen eingesetzt.

In den vergangenen Jahren sind bereits eine Reihe von Segelklappenprothesen bekannt geworden, bei welchen durch die verschiedensten Ausführungsformen versucht wurde, die natürliche Aorten-bzw. Pulmonalklappe in Funktion und Aufbau so gut wie möglich nachzuahmen. In der DE-PS 23 55 959 wird beispielsweise eine Taschenklappe zum Ersatz der Aorten-oder Pulmonalklappe beschrieben, die das wesentliche Konstruktionsmerkmal aufweist, daß sie in ihrem Querschnitt so flexibel ist, daß sie den Größen-und Querschnittsänderungen der Basis der Aorten-bzw. Pulmonalklappe zu folgen vermag. Ein solches sehr flexibles System hat zwar gewisse Vorteile, aber von der technischen Funktion her den Nachteil, daß die Segel bei starker Dehnung durchschlagen können. Auch läßt sich der textile Nahtring, mit welchem die Prothese im Herzen befestigt wird, nur schwierig an einem derart nachgiebigen Stützring fixieren.

Ein sehr wesentlicher Nachteil dieser und anderer bekannter Herzklappen besteht jedoch vor allem darin, daß die Höhe der Kommissurenstützen gleich der Höhe der Segelklappen ist, so daß die Kräfte, die bei der Öffnungs-und Schließbewegung der Segel auftreten, besonders an den Spitzen der Kommissurenstützen angreifen und zu einer Rißbildung an diesen belasteten Stellen führen können.

Aufgabe der Erfindung war es deshalb, eine Herzklappe der eingangs genannten Art zu entwickeln, welche diesen Schwachpunkt der bekannten prothetischen Herzklappen vermeidet. Außerdem sollte sie in ihren Konturen so geformt sein, daß sie keine Ansatzpunkte für Blutablagerungen, Thromben oder Verkalkungen bietet und sich mit großer Genauigkeit reproduzieren läßt durch die Einsatzmöglichkeit von möglichst computergesteuerten Maschinen bei der Formherstellung. Eine weitere Aufgabe der Erfindung war es, die bestgeeigneten Materialien, aus denen die Segel und der Stützring hergestellt werden können, aufzufinden sowie ein verbessertes Verfahren zu ihrer Herstellung zu entwickeln.

Gelöst wird die erstgenannte Aufgabe durch eine Herzklappenprothese zum Ersatz der Aorten-oder Pulmonalklappe aus einem Stützring mit mindestens je zwei Kommissurenstützen und flexiblen Segeln, die dadurch gekennzeichnet ist, daß die Höhe des Stützrings einschließlich der Kommissurenstützen kleiner als die Gesamthöhe der Herzklappenprothese ist. Vorzugsweise beträgt die genannte Höhe 20-80%, besonders vorteilhaft 40-60%, der Gesamthöhe.

Das bedeutet, daß der Stützring, welcher vorzugsweise aus einem Kunststoffmaterial besteht, das sich teilweise elastisch verformen läßt, nur den unteren Teil der Klappe abstützt, während der obere Teil der Segel und ihre Verbindungsbereiche - (die Kommissuren) frei und flexibel bleiben. Auf diese Weise wird erreicht, daß die Kräfte, die bei der Bewegung der Herzklappe konzentriert an den Spitzen der Kommissuren auftreten, sich harmonisch über die freie Höhe der Segelverbindungen verteilen können und nur ein Bruchteil direkt am Stützring angreift.

Um neben einer verlängerten Lebensdauer auch die strömungsdynamischen Eigenschaften der Herzklappe zu optimieren, besteht ein weiteres erfindungswesentliches Kennzeichen darin, daß alle ihre Konstruktionslinien und Flächen aus Geraden, Kreisen, Ebenen und Kugeln gebildet sind, deren Übergänge durch Rundungsradien angeglichen sind, so daß alle Kanten, Ecken und Übergänge gerundet sind, derart, daß die mathematischen Funktionen, die diesen Konstruktionslinien zugrunde liegen, stetig sind und sich vollständig differenzieren lassen.

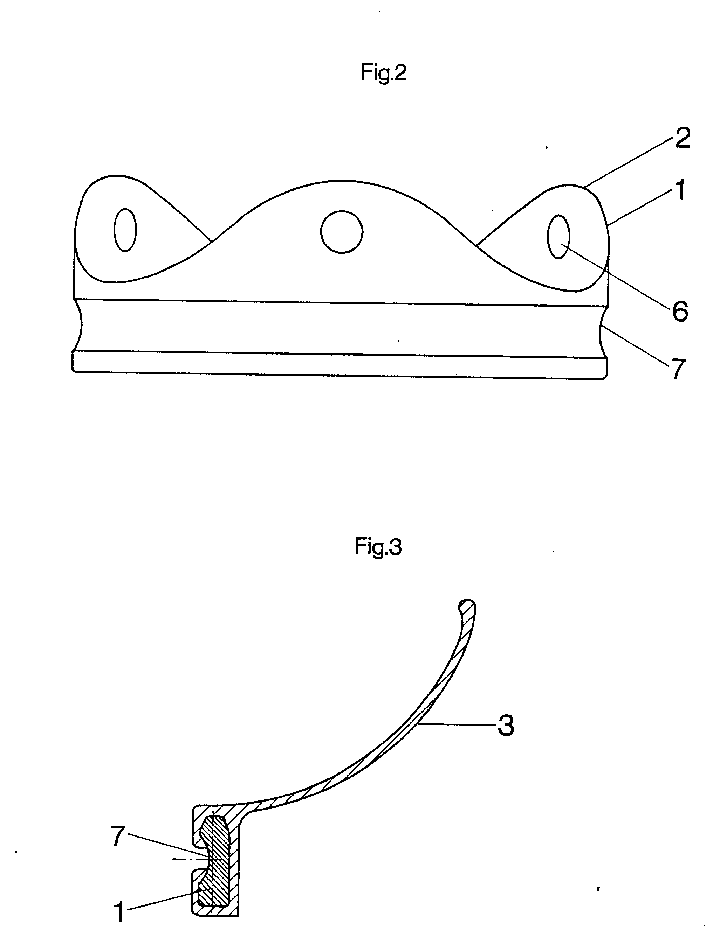

Die Formel für die Berechnung der geschwungenen Oberkante des Stützrings gemäß Figur 2 ist beispielsweise diesem Kriterium folgend, in Figur 4 wiedergegeben. Derartige mathematische Gleichungen und Funktionen lassen sich vom Fachmann berechnen und auch als Arbeitsprogramm in computergesteuerte Werkzeugmaschinen, die die Formen für die Herzklappenteileherstellung anfertigen, einspeichern.

Die weiteren vorzugsweisen Merkmale der erfindungsgemäßen Herzklappenprothese bestehen darin, daß sie drei flexible, nach innen gewölbte Segel aufweist, ähnlich der menschlichen Aortenklappe, und entsprechend drei Kommissurenstützen, wobei die Abschlußkanten der Segel in der Aufsicht die Kontur eines dreistrahligen symmetrischen Sterns zeigen, dessen Strahlen aus jeweils zwei benachbarten, parallel zueinander verlaufenden Abschlußkanten bestehen. Diese Abschlußkanten liegen auf einer gedachten Kugeloberfläche dergestalt, daß der Schnittpunkt der drei Kanten-Paare an der tiefsten Stelle liegt. Ferner sind die Abschlußkanten der Segel verdickt und abgerundet in der Weise, daß sie im Querschnitt keulenförmig ausgebildet sind. Dadurch weisen sie einen besonders niedrigen Strömungswiderstand für den Blutstrom und guten Einreißwiderstand auf.

Je nach der Shore-Härte und Reißfestigkeit des für die Segel verwendeten flexiblen Materials kann ihre Wandstärke 50-1000 micrometer betragen.

Entsprechend den angegebenen allgemeinen Konstruktionsmerkmalen, nach welchen alle Übergänge gerundet sind, ist auch der untere, innere Übergang der Segel an die Stützringkante abgerundet, was sich vorteilhaft auf die Stabilität und Belastbarkeit dieser, hoher Beanspruchung ausgesetzten Linie auswirkt.

Da die Kommissurenstützen des Herzklappenstützrings erfindungsgemäß nicht bis an die obere Kante der Kommissuren (Verbindungen) zwischen den Segeln reichen, bestehen diese in ihrem oberen Bereich aus dem Segelmaterial. Die Kommissuren sind vorteilhafterweise in diesem Bereich verstärkt, d.h. das Material ist hier verdickt, wodurch die Herzklappe mechanisch stabilisiert und gegen das Durchschlagen der Segel geschützt wird.

Der Stützring (oder "Stent"), wie er beispielsweise in Figur 2 wiedergegeben und der für drei Segel konzipiert ist, ist ein Ring mit einer ebenen unteren und einer geschwungenen oberen Kante sowie einer umlaufenden Nut für die Befestigung des Nahtrings. Die geschwungene Kante weist drei Stellen maximaler Höhe, die Kommissurenstützen, und drei Stellen minimaler Höhe auf, die jeweils radialsymmetrisch in einem Winkel von 120° angeordnet sind. Die umlaufende Nut und alle Kanten sind wiederum gerundet mit Ausnahme der eben aufliegenden unteren Innenkante. Im Bereich der drei höchsten Stellen befindet sich vorzugsweise je eine Öffnung, die vor allem zur Fixierung des Rings auf der Maschine bei der Herstellung der Herzklappe dient.

Der Stützring hat die Funktion, den flexiblen Segeln Halt zu geben und damit ein Durchschlagen der Klappe zu verhindern sowie dem Nahtring eine Verankerungsmöglichkeit zu bieten.

Für das Annähen der Herzklappe im Herzen können die üblichen textilen Nahtringe verwendet werden, die bevorzugt aus schlauchförmigen Geweben oder Gewirken aus Baumwolle-, Polyester-oder Polyamid-Fäden bestehen und in radialer Richtung dehnbar sind. Ein Nahtring aus einem Polyester(Polyethylenglycoltherephthalat)-Gewebe mit einer Wärmformbeständigkeit bis mindestens 150°C und einer radialen Dehnbarkeit von mindestens 10% ist besonders geeignet. Je nach Einsatz der Herzklappe werden verschiedene Nahtringe verwendet, vor allem die drei Versionen "subanular", "supraanular" oder "aortic". Die Befestigung des Nahtrings in der Nut des Stützringes kann durch Nähen, vorzugsweise durch Kleben erfolgen.

Ein weiteres vorzugsweises Merkmal, das vor allem zu einer möglichst langen Lebensdauer ohne Thrombosierung oder Verkalkung der erfindungsgemäßen Herzklappe beiträgt, besteht darin, daß der Stützring und die Segel einteilig ausgebildet sind indem die Kunststoffhaut, aus welcher die Segel gebildet sind, den Stützring mitumschließt. Die Nut am Stützring, in welcher der Nahtring befestigt ist, kann dabei ausgenommen sein. Auf diese Weise liegt eine Herzklappe wie "aus einem Guß" vor.

Um dies zu erreichen war es notwendig, ein besonders geeignetes Verfahren zur Herstellung der Prothese zu entwickeln und die bestgeeigneten Materialien zu finden.

Als Material für die Herstellung des Stützrings lassen sich die verschiedensten Produkte verwenden, sofern sie physiologisch unbedenklich sind und vom Körper nicht abgebaut werden. Beispielsweise sind dies Edelstahl, Titan, Niob, Tantal, Aluminium, Glaskohlenstoff, Quarzglas, Natronkalkglas, Calciumphosphatsinterkeramik, Titandioxid-sinterkeramik, Zirkondioxid-sinterkeramik oder thermoplastische Kunststoffe, wie Polyurethan, Polyethylenglycolterephthalat (Polyester), Polyethersulfon, Polycarbonat, Polyethylen oder Polypropylen mit einer Wärmeformbeständigkeit, die höher als 150°C, vorzugsweise zwischen 150 und 250°C liegt.

Als besonders geeignetes Material haben sich jedoch Polyamide erwiesen, die bis zu 50% ihres Gewichts kurze Glasfasern zur Verstärkung enthalten können. Das bevorzugte Polyamid-6,6 mit 15% Glasfasern läßt sich im Spritzguß verarbeiten und ist soweit hitzebeständig, um die notwendigen Temperaturen von 180-200°C kurzzeitig zu überstehen. Außerdem besitzt es hervorragende mechanische Eigenschaften, nämlich: Streckspannung (nach DIN 53 455) von größer als 60 N/mm², Reißdehnung (nach DIN 53 455) kleiner als 100%, Elastizitätsmodul (nach DIN 53 457) größer als 3000 N/mm², Dichte (nach DIN 53 497) größer als 1,1 g/m², Feuchtigkeitsaufnahme (nach DIN 53 479) kleiner als 3%, Wasseraufnahme bei 23°C - (nach DIN 53 495) kleiner als 9% und Formbeständigkeit (nach ISO 75, Verfahren A) größer als 150°C.

Bisher werden für flexible Herzklappen bevorzugt lineare, unvernetzte Polyetherurethane aus difunktionellen Isocyanaten, Alkoholen und Aminen verwendet, die entweder im Tauch-oder Tiefziehverfahren verarbeitet werden. Diese Urethane sind üblicherweise in Dimethylformamid, Dimethylacetamid oder in Dioxan/Tetrahydrofurangemischen gelöst und als 10-30%ige Lösungen im Handel.

Es hat sich jedoch in Tierversuchen gezeigt, daß sie im Organismus nicht vollkommen stabil gegen enzymatischen und/oder hydrolytischen Abbau sind. Der Grund mag darin liegen, daß die in den Polyurethanen enthaltenen Amidbindungen - NH-CO-vergleichbar mit den in den Eiweißstoffen vorhandenen Peptidbindungen sind.

Erfindunsgemäß wird dieses Problem dadurch gelöst, daß die flexiblen Segel aus einem dreidimensional vernetzten Polymer-Material bestehen, das sowohl in Wasser als auch in organischen Lösungsmitteln unlöslich ist. Wichtig dabei ist, daß das Material eine niedrige Shore-Härte A von 20-80 aufweist bei gleichzeitig hoher Reißfestigkeit.

Als besonders geeignet für diesen Zweck haben sich vernetzte Polyetherurethane erwiesen, die ein sogenanntes "Interpenetrating Network" (IPN) aufweisen. Dabei besteht der Vorteil, daß zur Herstellung der Segel im Tauchverfahren eine unvernetzte Lösung der Polymeren verwendet werden kann und die Vernetzung nach dem Trocknen des Films durch Behandeln mit Wasser erfolgt.

Als Polyetherurethane werden vorzugsweise segmentierte Polyetherurethane eingesetzt, wie sie beispielsweise in der US-PS 2,929,804 beschrieben sind. Diese Polyurethane haben sehr gute blutverträgliche Eigenschaften, sind aber im Gewebe abbaubar. Durch Zugabe von γ-Aminopropyltrisethoxysilan bzw. der entsprechenden Tri-methoxy-Verbindung lassen sich die Polyetherurethane jedoch nachträglich vernetzen. Die Vernetzung bewirkt ein Unlöslich-werden in Dimethylacetamid, in dem das unvernetzte Urethan kalt löslich ist. Zusätzlich kann der Lösung als Gleitmittel Isopropylmyristat zugefügt werden, um beim Tauchen ein besseres Verlaufen zu erreichen. Eine bevorzugte Mischung besteht aus 96% Urethan, welches durch mehrfaches Spülen in Essigsäureethylester von Schlichtemitteln und Verunreinigungen befreit wurde, 3% Isopropylmyristat und 1% γ-Aminopropyltrisethoxysilan. Derartige Polyurethane weisen nach der Vernetzungsreaktion eine Shore-Härte A von 60-80 auf. Die Wandstärke der Segel liegt mit diesem Material unter Verwendung des Tauchverfahrens bei 80-200 micrometer.

Ein weiteres besonders geeignetes Material ist Siliconkautschuk, hochtemperaturvernetzend ( =HTV ) und in der bevorzugten Form eines Zweikomponenten-Flüssigkautschukes ("Liquid Silicon Rubber=LSR") angewendet. Derartige Siliconkautschuke haben eine gute Blutverträglichkeit und Dauerwechselbiegefestigkeit sowie eine hohe Reißfestigkeit bei niedriger Shore-Härte A. Die bevorzugten Siliconkautschuke weisen nach der Vernetzung Shore-Härten A im Bereich von 25-60, vorzugsweise von 25-35, eine Reißfestigkeit von mindestens 8 N/mm², eine Zugfestigkeit von mehr als 8 MPa (nach DIN 53 504) und eine Bruchdehnung von mehr als 400% auf.

Sie lassen sich im Spritzguß verarbeiten und ihr äußerst günstiges Fließvermögen mit einer Viskosität von weniger als $2.10^6$ m Pa.s gestattet es, beim Formen bau mit geringsten Querschnitten bei Anguß-und Fließkanälen zu arbeiten. Da diese Siliconkautschuke, noch unvernetzt, auch in organischen Lösungsmitteln wie Hexan, Octan usw., Benzin, Toluol oder Xylol löslich sind, können sie wie die Polyurethane auch im Tauchverfahren verarbeitet werden.

Die Kautschuke werden gebildet durch die Reaktion von zwei linearen Polydimethylsiloxan-Komponenten, wobei die eine Komponente freie Vinylgruppen und die andere Si-H-Gruppen enthält. Beim Zusammentreffen reagieren diese beiden Gruppen in Form einer Additionsreaktion miteinander und verbinden und vernetzen dabei die beiden Komponenten. Als Katalysator für die Reaktion, die unter Wärmeeinwirkung abläuft, dienen organische Platinverbindungen.

Zur Verbesserung der mechanischen Eigenschaften können derartigen Siliconkautschuken obenflächenaktives Silicumdioxid bis zu etwa 25% zugesetzt werden.

Statt der flüssig zu verarbeitenden Siliconkautschuke können auch hochviskose 2-Komponenten-Siliconkautschuke verarbeitet werden. Diese müssen im Extruder vorgemischt werden und sind in ihren mechanischen Eigenschaften gleichwertig, jedoch ist die Verarbeitung aufwendiger.

Ziel des Herstellungsverfahrens für die erfindungsgemäßen Herzklappenprothesen war es, möglichst viele Herstellungsschritte maschinell und zudem vollautomatisch durchzuführen. Folgender Herstellungsablauf -in Stichworten aufgeführt - erfüllt diese Bedingungen:

1. Herstellen des Stützringes durch Spritzguß

2. Reinigen des Stützringes von Staubteilen, Fetten usw.

3. Haftungsförderndes Vorbehandeln des Stützrings (chemisch und/oder physikalisch) und gegebenenfalls Aufbringen einer haftungsvermittelnden Beschichtung

4. Gegebenenfalls ein-oder mehrfaches Tauchbeschichten des Stützrings mit dem Segelmaterial

5. Herstellen des flexiblen Segelteils durch Tauchbeschichten oder Spritzguß bei gleichzeitiger Anformung an den Stützring und Beschichten - (Umhüllen) des Stützrings mit dem Segelmaterial. Vernetzen des Segelmaterials.

6. Befestigen des textilen Nahtringes

7. Sterilisation der Herzklappe

8. Verpacken unter sterilen Bedingungen.

Erläuterung der einzelnen Teilschritte:

Wie bereits beschrieben, soll der Stützring vorzugsweise aus thermoplastischen Kunststoffen bestehen. Diese lassen sich ausgezeichnet im Spritzgußverfahren verarbeiten. Gegenüber den sogenannten "spanabhebenden" Formgebungstechniken hat der Spritzguß den Vorteil einer sehr genauen Reproduzierbarkeit, wenn die Form entsprechend genau gearbeitet ist und die Verfahrensparameter sorgfältig überwacht und eingehalten werden.

Selbstverständlich läßt sich der Stützring auch nach anderen Verfahren herstellen, diese stellen jedoch eine Verschlechterung gegenüber dem Spritzguß dar.

Durch den Verarbeitungsprozeß können sich auf dem Stützring Abriebpartikel, Spuren von Schmiermitteln usw. befinden. Diese werden vorzugsweise durch einen zweistufigen Waschprozeß beseitigt. Der erste Waschprozeß wird mit Wasser und Detergens durchgeführt, der zweite mit einem organischen Lösungsmittel bzw. Lösungsmittelgemisch. Hierfür eignen sich u.a. die bekannten organischen Lösungsmittel wie Aceton, Essigsäureethylester, Ethanol, Isopropanol, Trichlorethylen oder Benzin. Als oberflächenaktive Substanzen werden anionenaktive Verbindungen wie Natrium-laurylsulfonat bevorzugt.

Nach dem Trocknen wird der gereinigte Stützring zur besseren Haftung des Segelmaterials vorteilhafterweise vorbehandelt. Die Art der Behandlung hängt von den Materialien ab, aus denen der Stützring und die Segel bestehen. Für eine Kombination, bei welcher der Stützring aus Polyamid-6.6 mit 15% Glasfasern und die Segel aus Polyurethan bestehen, genügt es, das Polyamid mit Ameisensäure anzuätzen und mit Aceton zu waschen. Bei der Kombination dieses Stützrings mit Segeln aus Polydimethylsiloxan als flexibles Material, wird nach dem Ätzen eine haftungsvermittelnde Beschichtung vorgenommen. Wichtig ist, daß bei der fertigen Herzklappe die Adhäsion des flexiblen Materials auf dem Stützring größer ist als die Reißfestigkeit dieses Materials -d.h. Adhäsion größer als Kohäsion -, so daß es im Extremfall eher reißt als sich vom Stützring löst.

Erfindungsgemäß wird das Verfahren zur Herstellung der neuen Herzklappenprothese nach dem Tauchbeschichtungsverfahren mit einem Segelmaterial aus Polyurethan, d.h. in erster Linie aus den beschriebenen Polyetherurethanen, in der Weise durchgeführt, daß der gewaschene, geätzte und abgespülte Stützring mehrfach in eine ein Vernetzungsmittel enthaltende Polyurethanlösung getaucht (Lösungsmittel: Dimethylacetamid) und dazwischen jeweils soweit getrocknet wird, daß der Film nicht mehr fließt. Als Vernetzungsmittel eignen sich vorzugsweise das bereits erwähnte γ-Aminopropyltrisethoxy (oder trimethoxy)-silan. Nachdem eine Beschichtung von 30-50 micrometer Dicke erreicht ist, was in der Regel 3-5 Tauchvorgänge - (je nach der Viskosität und der Konzentration der Lösung) erfordert, wird der Ring über eine genau passende Tauchform der Segel geschoben, die so ausgearbeitet ist, daß die drei Segel mit Kommissuren in einem Stück gebildet werden, und dann das ganze Werkstück weiteren Tauchvorgängen unterworfen bis die gewünschte Filmdicke der Segel von 80-200 micrometer erreicht ist. Dies erfordert nochmals 8-20 Tauchvorgänge je nach Konzentration und Viskosität der Lösung.

Um eine gleichmäßige Schichtdicke zu erzielen, wird die Trocknung unter ständiger Rotation durchgeführt. Um die Oberfläche glatt und staubfrei zu halten, wird unter staubfreien "Laminar Air flow"-Bedingungen gearbeitet, wobei der Laminar-Strom vorgeheizt ist und die Lufttemperatur 50-60°C und die relative Luftfeuchtigkeit weniger als 25% beträgt. Die Trocknung zwischen den einzelnen Tauchvorgängen wird so geführt, daß das Urethan nicht mehr fließt und die neue Schicht die alte anquillt, aber nicht mehr zerstört. Dies geschieht dadurch, daß das Lösungsmittel nicht vollständig entfernt wird sondern nur jeweils bis zu 95%. Nachdem die erwünschte Schichtstärke erreicht ist, wird 24 Stunden bei 80°C nachgetrocknet, sodann entformt und anschließend 100 Stunden in destilliertem oder entionisiertem Wasser bei 80°C die Vernetzung durchgeführt, gleichzeitig werden dabei Lösungsmittelreste und andere lösliche Hilfsstoffe extrahiert.

Wird der flexible Teil auf diese Weise hergestellt, erfolgt die Formgebung des wulstartigen Abschlusses der Segelkanten durch "Schmelzschneiden" entweder mit einem Heizdraht oder einem Laserstrahl.

Dieses Verfahren zur Herstellung der Herzklappenprothese unterscheidet sich von dem in der DE-PS 32 48 560 beschriebenen u.a. vorteilhaft dadurch, daß zuerst der Stützring für sich getaucht wird und dadurch allseitig beschichtet ist bevor er über die Tauchform für die Segel geschoben und mit dieser zusammen weiter beschichtet wird. Dies ergibt einen besonders glatten Übergang vom Stützring zu den Segeln. Außerdem entsteht durch die nur unvollständige Trocknung der Polymerfilme zwischen den einzelnen Tauchvorgängen ein fester und homogener Verbund in der Beschichtung.

Das Verfahren zur Herstellung der Herzklappenprothese mit Segeln aus Siliconkautschuk nach dem Reaktionsspritzgußverfahren (Liquid-Silicon-Rubber = LSR-Spritzguß) wird in der Weise durchgeführt, daß der mit einer geeigneten Verankerungsschicht vorbehandelte Stützring in die Form eingelegt und über Stifte darin fixiert wird, die beiden Siliconkomponenten über eine Dosieranlage in ein Mischrohr (statischer Mischer) gefördert werden, dort zusammen mit dem Katalysator und gegebenenfalls weiteren Zusatzstoffen wie Siliciumdioxid blasenfrei vermischt werden und dann über eine Nadeldüse in die Form eingespritzt werden. Die Vulkanisation erfolgt anschließend in der Form bei 180°C während 10 Minuten. Niedrigere Temperaturen können angewendet werden, wenn die Reaktionszeit entsprechend verlängert wird, jedoch darf eine minimale Reaktionstemperatur von 80°C nicht unterschritten werden, wenn die mechanischen Eigenschaften nicht beeinträchtigt werden sollen.

Um eine reproduzierbare und GMP-(Good Manufacturing Practice)-gerechte Fertigung sicherzustellen, sollten Temperatur und Druck innerhalb der Form während der Vulkanisationszeit überwacht und aufgezeichnet werden und ständig ein Soll-Ist-Vergleich erfolgen. Voraussetzung für ein solches Verfahren ist eine Spritzgußform mit einer Fertigungstoleranz von 7-3 micrometer und einer Rauhtiefe von 5-3 micrometer. Außerdem müssen die Formtrennebenen außerhalb der Blutströmungsrichtung liegen.

Die Spritzgußform ist dabei so ausgestaltet, daß beim Spritzgießen neben der Ausbildung der Segel gleichzeitig auch ein Ummanteln des Stützrings mit der Siliconkautschuk-Schicht stattfindet. Die abgerundeten Abschlußkanten der Segel werden bei diesem Verfahren durch entsprechende Formgebung erzeugt.

Nach der Herstellung der Herzklappe wird der Nahtring an der Prothese wie üblich durch Vernähen und/oder Verkleben in der umlaufenden Nut im Stützring befestigt.

Die erfindungsgemäßen Herzklappenprothesen, die sich durch ihre besondere Formgestaltung, die bevorzugt verwendeten Materialien und eine darauf abgestimmte optimierte Herstellung auszeichnen, weisen ein besonders günstiges strömungstechnisches Verhalten, eine hohe mechanische Stabilität und Dauerbeständigkeit sowie eine gute Blutverträglichkeit auf.

Nachstehend wird die Herzklappenprothese anhand der Abbildungen beispielsweise erläutert:

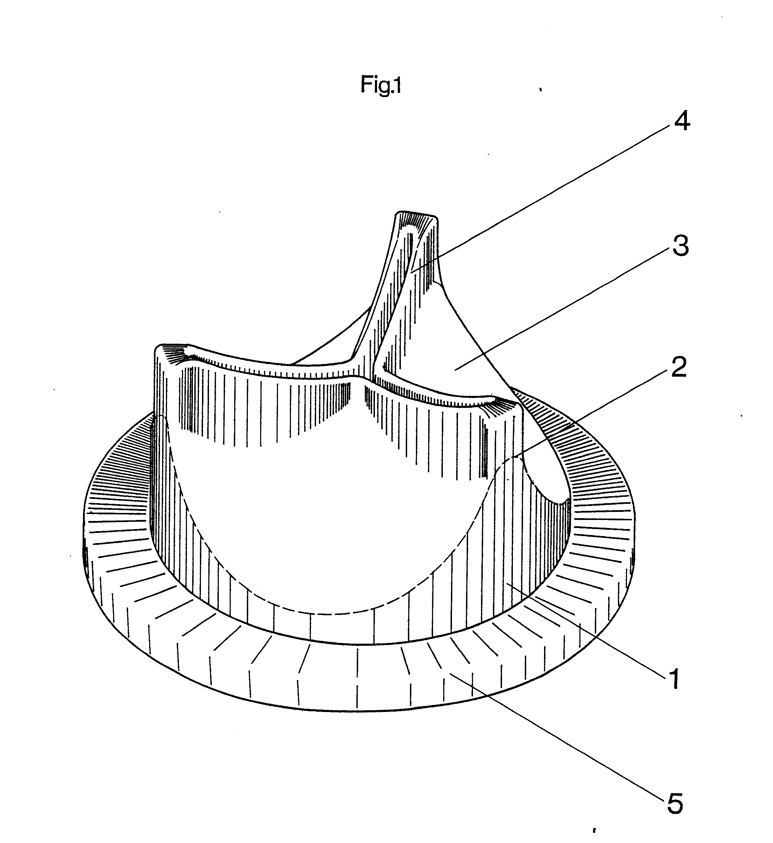

Figur 1 zeigt eine erfindungsgemäße Herzklappe in perspektivischer Ansicht mit dem Stützring 1, den Kommissurenstützen 2, den drei Segeln 3, den Segelabschlußkanten 4 und dem Nahtring 5. Der Stützring ist dabei nur durch unterbrochene Linien angezeigt, da er nicht frei liegt sondern mit dem flexiblen Segelmaterial überzogen ist.

Figur 2 zeigt den freien Stützring 1 mit den Kommissurenstützen 2, den Öffnungen 6 und der umlaufenden Nut 7 für die Befestigung des Nahtringes.

Figur 3 zeigt einen Teil-Querschnitt durch die Herzklappe mit einem Segel 3, dem Stützring 1 und der Nut 7. Hierbei ist insbesondere ersichtlich, wie das Segelmaterial den Stützring umschließt und daß die Abschlußkante des dargestellten Segels eine gerundete, keulenförmig verdickte Kontur aufweist.

Figur 4 gibt die Formel zur Berechnung der Oberkante des Stützrings wieder.

**Ansprüche**

1) Herzklappenprothese zum Ersatz der Aorten-oder Pulmonalklappe aus einem Stützring mit mindestens je zwei Kommissurenstützen und flexiblen Segeln, dadurch gekennzeichnet, daß die Höhe des Stützrings einschließlich der Kommissurenstützen kleiner als die Gesamthöhe der Herzklappenprothese ist.

2) Herzklappenprothese gemäß Anspruch 1, dadurch gekennzeichnet, daß die Höhe des Stützrings einschließlich der Kommissurenstützen 20-80% der Gesamthöhe der Herzklappenprothese beträgt.

3) Herzklappenprothese gemäß Anspruch 1, dadurch gekennzeichnet, daß alle Konstruktionslinien und Flächen aus Geraden, Kreisen, Ebenen und Kugeln gebildet sind, deren Übergänge durch Rundungsradien angeglichen sind, so daß alle Kanten, Ecken und Übergänge gerundet sind, derart, daß die mathematischen Funktionen, die diesen Konstruktionslinien zugrunde liegen, stetig sind und vollständig differenzierbar sind.

4) Herzklappenprothese gemäß Anspruch 1, dadurch gekennzeichnet, daß sie drei flexible, nach innen gewölbte Segel und drei Kommissurenstützen aufweist und die Abschlußkanten der Segel in der Aufsicht die Kontur eines dreistrahligen symmetrischen Sterns zeigen, dessen Strahlen aus jeweils zwei benach barten parallel zueinander verlaufenden Abschlußkanten bestehen.

5) Herzklappenprothese gemäß Anspruch 1, dadurch gekennzeichnet, daß die Abschlußkanten der Segel auf einer gedachten Kugeloberfläche liegen, dergestalt, daß der Schnittpunkt der drei Segelkanten-Paare an der tiefsten Stelle liegt.

6) Herzklappenprothese gemäß Anspruch 1, dadurch gekennzeichnet, daß die Abschlußkanten der Segel gerundet sind und im Querschnitt eine keulenförmige Kontur aufweisen.

7) Herzklappenprothese gemäß Anspruch 1, dadurch gekennzeichnet, daß der Stützring und die Segel einteilig ausgebildet sind, indem die Kunststoffhaut, aus welcher die Segel gebildet sind, den Stützring mitumschließt.

8) Herzklappenprothese gemäß Anspruch 1, dadurch gekennzeichnet, daß der Stützring aus Edelstahl, Titan, Niob, Tantal, Aluminium, Glaskohlenstoff, Quarzglas, Silikatglas, Calciumphosphat-sinterkeramik, Titandioxid-sinterkeramik, Zirkoniumdioxid-sinterkeramik oder aus einem thermoplastischen Kunststoff, dessen Wärmeformbeständigkeit höher als 150°C, vorzugsweise zwischen 150 und 250°C liegt, besteht.

9) Herzklappenprothese gemäß Anspruch 1, dadurch gekennzeichnet, daß der Stützring aus Polyamid-6, Polyamid-6,6, Polyamid-6,10, Polyamid-12, Polyethylenglycolterephthalat (Polyester), Polyurethan, Polyethersulfon, Polyoxymethylen oder Polycarbonat, gegebenenfalls mit bis zu 50 Gew.-% Glasfasern, besteht.

10) Herzklappenprothese gemäß Anspruch 1, dadurch gekennzeichnet, daß der Stützring aus Polyamid-6,6 mit 15% Glasfasern besteht.

11) Herzklappenprothese gemäß Anspruch 1, dadurch gekennzeichnet, daß die Segel aus einem dreidimensional vernetzten flexiblen Material bestehen, das in allen organischen Lösungsmitteln und Wasser unlöslich ist und eine Shore-Härte A von 20-80 aufweist.

12) Herzklappenprothese gemäß Anspruch 1, dadurch gekennzeichnet, daß die Segel aus einem unlöslichen, mit γ-Amino-propyl-tris-alkoxy-silan vernetzten Polyetherurethan mit einer Shore-Härte A von 60-80 bestehen.

13) Herzklappenprothese gemäß Anspruch 1, dadurch gekennzeichnet, daß die Segel aus einem dreidimensional hochtemperatur-vernetzten Polydimethylsiloxan, welches bis zu 25% aktives Siliciumdioxid enthalten kann, mit einer Shore-Härte A von 25-60 und einer Mindestreißfestigkeit von 8 N/mm² bestehen.

14) Verfahren zur Herstellung einer Herzklappenprothese mit einem Segelmaterial aus Polyurethan nach dem Tauchbeschichtungsverfahren, dadurch gekennzeichnet, daß zuerst der gegebenenfalls haftungsfördernd vorbehandelte Stützring mit einer PolyurethanBeschichtung versehen wird, dann dieser Stützring über die Tauchform der Segel geschoben wird und mit dieser gemeinsam ein oder mehrmals in der ein Vernetzungsmittel enthaltenden Polyurethanlösung tauchbeschichtet wird, wobei zwischen den einzelnen Tauchvorgängen derart getrocknet wird, daß das Lösungsmittel nicht ganz entfernt wird, sondern maximal zu 95% und erst nach dem letzten Tauchvorgang vollständig getrocknet wird, und anschließend die Vernetzung durchgeführt wird, indem 24 bis 100 Stunden bei ca. 80°C in Wasser erhitzt wird.

15) Verfahren zur Herstellung einer Herzklappenprothese mit Segeln aus Siliconkautschuk, dadurch gekennzeichnet, daß nach dem Reaktionsspritzgußverfahren mit flüssigen Siliconen gearbeitet wird, wobei zuerst der haftungsfördernd vorbehandelte Stützring in der Form fixiert wird, dann die mit dem Katalysator und gegebenenfalls weiteren Zusatzstoffen vermischten flüssigen Siliconkomponenten in die Form blasenfrei eingespritzt werden und anschließend die Vernetzung des Siliconkautschuks in der Form bei 100-200°C erfolgt.

Fig.1

Fig.2

Fig.3

## Fig.4

## Formel zur Berechnung der Stützringoberkante

$$F(x) = \frac{a_0}{2} + \sum_{K=1}^{K=\infty} \left\{ \left[ \frac{2}{\ddot{U}_1} \cdot \int_0^{\ddot{U}_1} \left( \frac{h_{st}}{2} \cdot \cos(x) + \frac{h_{st}}{2} \right) \cdot \cos(K \cdot \omega_0 \cdot x) \cdot dx \right] \right.$$

$$+ \left[ \frac{2}{\ddot{U}_1} \cdot \int_0^{\ddot{U}_1} \left( \frac{h_{st}}{2} \cdot \cos(x) + \frac{h_{st}}{2} \right) \cdot \sin(K \cdot \omega_0 \cdot x) \cdot dx \right]$$

$$+ \left[ \frac{2}{\ddot{U}_2 - \ddot{U}_1} \cdot \int_{\ddot{U}_1}^{\ddot{U}_2} \left( r_3 - \sqrt{r_3^2 - r_2^2 - r_1^2 + 2 \cdot r_2 \cdot r_1 \cdot \cos\left( \frac{\delta \cdot \pi}{180} \right)} \right) \cdot \cos(K \cdot \omega_0 \cdot x) \cdot dx \right]$$

$$+ \left[ \frac{2}{\ddot{U}_2 - \ddot{U}_1} \cdot \int_{\ddot{U}_1}^{\ddot{U}_2} \left( r_3 - \sqrt{r_3^2 - r_2^2 - r_1^2 + 2 \cdot r_2 \cdot r_1 \cdot \cos\left( \frac{\delta \cdot \pi}{180} \right)} \right) \cdot \sin(K \cdot \omega_0 \cdot x) \cdot dx \right]$$

$$+ \left[ \frac{2}{T_0 - \ddot{U}_2} \cdot \int_{\ddot{U}_2}^{T_0} \left( \frac{h_{st}}{2} \cdot \cos(x) + \frac{h_{st}}{2} \right) \cdot \cos(K \cdot \omega_0 \cdot x) \cdot dx \right]$$

$$\left. + \left[ \frac{2}{T_0 - \ddot{U}_2} \cdot \int_{\ddot{U}_2}^{T_0} \left( \frac{h_{st}}{2} \cdot \cos(x) + \frac{h_{st}}{2} \right) \cdot \sin(K \cdot \omega_0 \cdot x) \cdot dx \right] \right\}$$

$\ddot{U}_1 = $ Übergabepunkt 1

$\ddot{U}_2 = $ Übergabepunkt 2

$T_0 = $ Periodenlänge = Stützringumfang $/3$

$h_{st} = $ maximale Stützringhöhe

$\omega_0 = 2 \cdot \pi / T_0$

$r_1 = $ Stützringaußenradius

$r_2 = $ Stützringmittelradius

$r_3 = $ Konstruktionskugelradius

$\delta \quad = $ Konstruktionswinkel

$K \quad = $ Laufvariable

$x \quad = $ Position auf der Stützringabwicklung

$\emptyset \leqslant x \leqslant $ Stützringumfang $/3$

$a_0 = $ Konstruktive, radiusabhängige Hilfsgröße